# FASCICULE DE BREVET EUROPEEN

(11) **EP 1 296 667 B1**
(45) Date de publication et mention de la délivrance du brevet: **01.03.2006**
(21) Numéro de dépôt: 01940621.4
(22) Date de dépôt: 29.05.2001
(51) Int. Cl.: A61K 31/198, A61K 33/06, A61P 17/00

(54) **COMPOSES POUR LA PREVENTION ET/OU LE TRAITEMENT DES URTICAIRES**
VERBINDUNGEN ZUR PROPHYLAXE UND/ODER BEHANDLUNG VON NESSELSUCHT
COMPOUNDS FOR PREVENTING AND/OR TREATING URTICARIA

(30) Priorité: 07.06.2000 FR 0007309
(43) Date de publication de la demande: 02.04.2003
(73) Titulaire: Meyer, Marie-Claude, 75015 Paris (FR)
(72) Inventeur: MEYER, Marie-Claude, F-75015 Paris (FR)
(74) Mandataire: Touati, Catherine
(86) Numéro de dépôt international: PCT/FR2001/001657
(87) Numéro de publication internationale: WO 2001/093858

(56) Documents cités:
- US-A- 4 857 321
- DATABASE WPI Section Ch, Week 199139 Derwent Publications Ltd., London, GB; Class A96, AN 1991-284799 XP002161898 & JP 03 188149 A (OTSUKA SEIYAKU KOGYO KK) , 16 août 1991 (1991-08-16)
- FAGIOLO, UMBERTO ET AL: "Inhibitory effect of heparin on skin reactivity to autologous serum in chronic idiopathic urticaria" J. ALLERGY CLIN. IMMUNOL. (1999), 103(6), 1143-1147, XP000986956
- KAPLAN A.P. ET AL: "In vivo studies of the pathogenesis of cold urticaria, cholinergic urticaria, and vibration induced swelling." JOURNAL OF INVESTIGATIVE DERMATOLOGY, (1976) 67/3 (327-332). CODEN: JIDEAE, XP000986947
- GRAFF L ET AL: "In vitro and in vivo evaluation of potential aluminum chelators." VETERINARY AND HUMAN TOXICOLOGY, (1995 OCT) 37 (5) 455-61., XP000986952
- GUTTERIDGE J M ET AL: "Aluminium salts accelerate peroxidation of membrane lipids stimulated by iron salts." BIOCHIMICA ET BIOPHYSICA ACTA, (1985 JUL 31) 835 (3) 441-7., XP000984109
- KONTOGHIORGHES G.J.: "Comparative efficacy and toxicity of desferrioxamine, deferiprone and other iron and aluminium chelating drugs." TOXICOLOGY LETTERS, (1995) 80/1-3 (1-18)., XP000986910

## Description

La présente invention porte sur l'utilisation d'un complexe formé entre l'ion Al³⁺ et le ligand éthylènediamine-tétraacétique (EDTA) pour la fabrication d'un produit destiné à la prévention et/ou au traitement des urticaires.

Les urticaires sont des éruptions cutanées, souvent passagères, se caractérisant par des enflures prurigineuses de diverses grandeurs. Une fois-disparues, les éruptions ne laissent aucune trace sur la peau et il n'y a pas de desquamation. L'urticaire peut parfois atteindre les tissus plus profonds, on parle alors d'angioderme.

Les urticaires peuvent être classées en deux grandes catégories, les urticaires allergiques et les urticaires non allergiques. On classe parmi les urticaires non allergiques, par exemple les urticaires physiques dues à l'action du soleil, du froid, de la chaleur ou des frottements, et les urticaires secondaires qui sont associées à une maladie auto-immune, une thyroiodopathie ou une infection parasitaire.

Dans le cas des urticaires allergiques, l'inflammation apparaît en général moins de 30 minutes après le contact avec l'agent sensibilisant. Cet agent sensibilisant peut être un aliment, la piqûre d'un insecte tel qu'une abeille, un bourdon, une araignée, certains animaux tels que la méduse, certains végétaux tels que l'ortie, le mancellinier ou arbre de mort de Martinique, le sumac vénéneux plus connu sous le nom de "poison ivy".

Le mécanisme d'action de ces divers agents sensibilisants est encore assez mal connu.

Dans le cas des méduses, le moindre contact entre les tentacules et la peau entraîne une sensation de brûlure intense. En fait, une toxine présente dans les tentacules est injectée au niveau de la peau et provoque un blocage de l'influx nerveux, un éclatement des globules rouges du sang et une réaction de défense par production d'anticorps.

L'effet du venin est proportionnel à la surface touchée du corps. C'est pourquoi les enfants présentent des réactions beaucoup plus violentes que les adultes.

Classiquement, les urticaires, notamment les urticaires allergiques, sont traitées à l'aide de produits tels que des analgésiques, des anti-inflammatoires ou des antihistaminiques, qui sont principalement administrés par voie orale ou par injection, et plus rarement par application topique.

Ces produits sont actifs sur les manifestations physiologiques provoquées par les produits urticants, c'est à dire qu'ils soulagent, éventuellement ralentissent notamment l'inflammation, mais n'interviennent pas sur les causes et n'arrêtent pas le processus de développement des manifestations telles que l'inflammation, la rougeur et la douleur. Par ailleurs, lorsqu'ils sont administrés par voie orale, ils provoquent inévitablement des effets secondaires plus ou moins prononcés.

De façon inattendue et surprenante, les inventeurs ont trouvé qu'il était possible de stopper l'action des substances urticantes par l'application d'un complexe formé entre l'ion aluminium Al³⁺ et le ligand éthylènediamine-tétraacétique sur la partie de la peau qui est entrée en contact avec l'agent urticant.

Le brevet JP 03 188 149 (OTSUKA SEIYAKU KOGYO KK) décrit un gel pour la protection de la peau. Ce gel est utilisé pour la protection de patients contre les brûlures, les blessures, les piqûres d'insectes, les brûlures du soleil et les dermatites purulentes.

De même, le brevet US 4 857 321 (THOMAS William), décrit une pommade pour calmer les atteintes à la peau et permettre son humidification. Les atteintes à la peau calmées sont les brûlures le psoriasis, le pied d'athlète, l'acné, l'érythème fessier du bébé, les escarres et les piqûres d'insectes.

Bien que décrivant dans une même composition la présence d'ions Al³⁺ et d'EDTA, ces documents ne décrivent toutefois ni la formation des complexes de l'invention, ni le traitement de l'urticaire.

La présente invention porte donc sur l'utilisation d'une quantité efficace d'un complexe formé entre l'ion aluminium Al³⁺ et un ligand éthylènediamine-tétraacétique (Y) choisi dans le groupe comprenant les complexes AlY, AlHY, Al (OH) Y, Al(OH)₂Y et leurs mélanges, pour la fabrication d'un produit destiné à la prévention et/ou au traitement des urticaires.

Les complexes peuvent être associés à des cations dont notamment le Na⁺.

Le complexe est présent dans le produit à une concentration de 0,05 mole/l à 1,5 mole/l, de préférence de 0,1 mole/l à 1 mole/l, et plus préférentiellement encore de 0,1 à 0,4 mole/l.

Selon un mode de réalisation avantageux, le complexe est Al(OH)Y et il est utilisé à une concentration de 0,05mole/l à 1,5 mole/l, de préférence de 0,1 à 0,6 mole/l, et plus préférentiellement encore de 0,1 à 0,4 mole/l.

Conformément à l'invention, le complexe est utilisé pour la fabrication d'un produit destiné à une application topique, plus particulièrement cutanée. Cependant, si l'agent urticant est entré en contact avec les yeux, il peut être utilisé comme bain oculaire et, si l'agent urticant est entré en contact avec la muqueuse buccale, il peut également être utilisé comme bain de bouche en prenant soin d'éviter toute ingestion.

Afin de permettre une application topique, le produit peut se présenter sous la forme d'une solution ou d'une dispersion, d'une pommade, d'un gel, d'un spray, de lotions, de compresses, de tampons, de pansements.

Dans le cas des pansements, compresses et tampons, il s'agit de supports imprégnés dudit produit, ledit substance produit se libérant lorsque le support est appliqué sur la peau.

Selon un mode de réalisation avantageux, le produit comprend au moins un excipient choisi dans le groupe comprenant notamment des agents apaisants, des agents rafraîchissants, des parfums, des agents colorants, des ampholytes, du chlorure de sodium, des sucres, de la glycérine, des glycols, des solvants, des agents épaississants, des agents conservateurs.

A titre d'exemples d'agents apaisants, on peut citer ceux choisis dans le groupe comprenant notamment l'allantoïne, l'eau de bleuet et l'eau de rose.

A titre d'exemples d'agents rafraîchissants, on peut citer ceux choisis dans le groupe comprenant notamment le menthol et l'essence d'eucalyptus.

La présence d'agents colorants est particulièrement avantageuse dans le cas où le produit se présente sous la forme d'une pommade ou d'un gel, car elle permet de visualiser la zone d'application du produit.

On peut citer parmi les ampholytes pouvant entrer dans la composition du produit, le citrate disodique, l'alanine, le glutamate disodique, le glycocolle, la lysine, l'alanyl-alanine, le bicarbonate de sodium, l'édétate trisodique (sel trisodique de l'EDTA).

Dans le cas où on utilise du glucose pour la fabrication du produit, sa concentration est choisie de manière à ajuster à une valeur donnée l'isotonicité ou l'hypertonicité du produit selon les applications et les parties du corps à traiter.

Des solvants, des glycols ou de la glycérine peuvent être ajoutés de façon à améliorer la solubilisation du complexe si nécessaire.

Selon un mode de réalisation particulièrement avantageux, lorsque le produit se présente sous la forme d'une solution aqueuse, son pH est compris entre 6 et 9.

L'utilisation conforme à l'invention est particulièrement adaptée à la prévention et/ou au traitement des urticaires provoquées par les agents urticants choisis dans le groupe comprenant notamment les méduses, les orties, le mancellinier, le sumac vénéneux, les piqûres d'insectes.

Pour une efficacité optimale, il est souhaitable que le produit soit appliqué le plus rapidement possible après le contact de la peau avec l'agent urticant, de préférence dans la demi-heure suivant ce contact, et qu'il soit éventuellement appliqué une seconde fois à un intervalle d'application de 30 minutes à 2 heures.

Bien entendu, plus l'application est tardive, plus les symptômes de l'urticaire sont développés, notamment l'inflammation, mais le produit utilisé permet d'éliminer l'agent urticant qui reste toujours en surface. Dans le cas d'une application précoce, l'action de l'agent urticant est stoppée, et les manifestations physiologiques sont alors très réduites, voire invisibles.

L'invention va maintenant être décrite plus en détail à l'aide de l'exemple suivant.

### EXEMPLE

On a utilisé un spray présentant la composition suivante
Complexe Al(OH)Y à une concentration de 0,3 mole/l
Excipient aqueux qsp 200ml
pH 7,4

Sur 64 patients piqués par des méduses on a appliqué sur la zone de la peau concernée le spray ci-dessus.

Cette application a été faite plus ou moins longtemps après la piqûre mais dans tous les cas dans l'heure qui a suivi la piqûre.

Pour chaque patient on a regardé quelques minutes après l'application l'effet sur l'oedème, la rougeur, l'aspect général de la peau.

L'effet sur l'oedème et l'effet sur la rougeur ont été qualifiés de la façon suivante:
0 : aucun effet
1 : légère diminution
2 : diminution importante
3 : disparition complète

L'effet sur l'aspect général a été qualifié de la façon suivante:
0 : aucun effet, aspect général non modifié
1 : légère amélioration
2 : amélioration importante
3 : parfait, plus aucune trace de l'urticaire.

Chaque patient a également indiqué l'effet obtenu sur la douleur en utilisant la même caractérisation que précédemment.

Les résultats sont rassemblés dans le tableau ci-après.

Ils sont exprimés en pourcentage calculés sur la totalité des 64 patients.

| | Oedème | Rougeur | Aspect général | Douleur |
|---|---|---|---|---|
| Aucun effet | 6 | 3 | 3 | 3 |
| léger | 8 | 42 | 17 | 6 |
| Effet important | 59 | 44 | 69 | 33 |
| Disparition | 27 | 11 | 11 | 58 |

Il ressort de cet exemple que, pour la majorité des patients (80%), l'application du complexe a eu pour résultat une amélioration importante de l'aspect général de la zone qui présentait initialement l'urticaire, et ceci seulement quelques minutes après l'application. Par ailleurs, il est intéressant de noter que la douleur a complètement disparu quelques minutes après l'application chez 58% des patients et qu'elle a diminué de façon importante chez 33% des patients.

## Revendications

1. Utilisation d'une quantité efficace d'un complexe formé entre l'ion aluminium Al³⁺ et un ligand éthylènediamine-tétraacétique (Y) choisi dans le groupe comprenant les complexes AlY, AlHY, Al(OH)Y, Al(OH)₂Y et leurs mélanges, pour la fabrication d'un produit destiné à la prévention et/ou au traitement des urticaires par application topique.

2. Utilisation selon la revendication 1, **caractérisée par le fait que** le complexe est présent dans le produit dans une concentration de 0,05 mole/l à 1,5 mole/l, de préférence de 0,1 mole/l à 1 mole/l, et plus préférentiellement encore de 0,1 à 0,4 mole/l.

3. Utilisation selon la revendication 1 ou la revendication 2, caractérsisée par le fait que le complexe est Al(OH)Y et que sa concentration est de 0,05mole/l à 1,5 mole/l, de préférence de 0,1 à 0,6 mole/l, et plus préférentiellement encore de 0,1 à 0,4 mole/l.

4. Utilisation selon l'une quelconque des revendications 1 à 3, **caractérisée par le fait que** le produit se présente sous la forme d'une solution ou d'une dispersion, d'une pommade, d'un gel, d'un spray, de compresses, de lotions, de pansements.

5. Utilisation selon l'une quelconque des revendications 1 à 4, **caractérisée par le fait que** le produit comprend au moins un excipient choisi dans le groupe comprenant des agents apaisants, des agents rafraîchissants, des parfums, des agents colorants, des ampholytes, du chlorure de sodium, des sucres, de la glycérine, des glycols, des solvants, des agents épaississants.

6. Utilisation selon l'une quelconque des revendications 1 à 5, **caractérisée par le fait que** le produit se présente sous la forme d'une solution aqueuse présentant un pH compris entre 6 et 9.

7. Utilisation selon l'une quelconque des revendications 1 à 6, pour la prévention et/ou le traitement des urticaires provoquées par les agents urticants choisis dans le groupe comprenant notamment les méduses, les orties, le mancellinier, le sumac vénéveux, les piqûres d'insectes.

## Claims

1. Use of an effective amount of a complex formed between the aluminium ion Al³⁺ and an ethylenediaminetetraacetic ligand (Y) chosen from the group consisting of the complexes AlY, AlHY, Al(OH)Y, Al(OH)₂Y and their mixtures in the manufacture of a product intended for the prevention and/or treatment, by topical application, of urticaria.

2. Use according to Claim 1, **characterized in that** the complex is present in the product in a concentration of 0.05 mol/l to 1.5 mol/l, preferably of 0.1 mol/l to 1 mol/l and more preferably still of 0.1 to 0.4 mol/l.

3. Use according to Claim 1 or Claim 2, **characterized in that** the complex is Al(OH)Y and **in that** its concentration is from 0.05 mol/l to 1.5 mol/l, preferably from 0.1 to 0.6 mol/l and more preferably still from 0.1 to 0.4 mol/l.

4. Use according to any one of Claims 1 to 3, **characterized in that** the product is provided in the form of a solution or of a dispersion, of an ointment, of an gel, of a spray, of compresses, of lotions or of dressings.

5. Use according to any one of Claims 1 to 4, **characterized in that** the product comprises at least one excipient chosen from the group consisting of soothing agents, cooling agents, fragrances, colouring agents, ampholytes, sodium chloride, sugars, glycerol, glycols, solvents and thickening agents.

6. Use according to any one of Claims 1 to 5, **characterized in that** the product is provided in the form of an aqueous solution exhibiting a pH of between 6 and 9.

7. Use according to any one of Claims 1 to 6 in the prevention and/or treatment of urticaria brought about by urticants chosen from the group consisting in particular of jellyfish, nettles, manchineel, poison ivy and insect stings.

## Patentansprüche

1. Verwendung einer wirksamen Menge eines Komplexes, der zwischen einem Aluminiumion Al³⁺ und einem Liganden Ethylendiamintetraacetat (Y) gebildet wird, das aus der die Komplexe AlY, AlHY, Al(OH)Y, Al(OH)₂Y und ihre Mischungen enthaltenden Gruppe gewählt wird, zur Herstellung eines Produkts, das durch topische Anwendung der Prävention und/oder der Behandlung von Nesselsacht zu dienen bestimmt ist.

2. Verwendung nach Anspruch 1, **dadurch gekennzeichnet, dass** der Komplex in dem Produkt in einer Konzentration von 0,05 mol/l bis zu 1,5 mol/l, vorzugsweise von 0,1 mol/l bis 1 mol/l, und noch bevorzugterweise von 0,1 bis 0,4 mol/l, vorliegt.

3. Verwendung nach Anspruch 1 oder 2, **dadurch gekennzeichnet, dass** der Komplex AI(OH)Y ist und seine Konzentration zwischen 0,05 mol/l bis 1,5 mol/l, vorzugsweise zwischen 0,1 bis 0,6 mol/l, und noch bevorzugterweise zwischen 0,1 bis 0,4 mol/l, beträgt.

4. Verwendung nach einem der vorangegangenen Ansprüche 1 bis 3, **dadurch gekennzeichnet, dass** das Produkt in einer Lösung oder einer Dispersion, einer Creme, einem Gel, einem Spray, Kompressen, Lotionen oder Verbänden dargereicht wird.

5. Verwendung nach einem der vorangegangenen Ansprüche 1 bis 4, **dadurch gekennzeichnet, dass** das Produkt wenigstens ein Hilfsstoff enthält, das aus der Gruppe gewählt wird, die Beruhigungsmittel, Erfrischungsmittel, Parfums, Färbemittel, Ampholyte, Natriumchlorid, Zucker, Glyzerin, Glykole, Lösungsmittel und Verdickungsmittel enthält.

6. Verwendung nach einem der vorangegangenen Ansprüche 1 bis 5, **dadurch gekennzeichnet, dass** das Produkt in Form einer wässrigen Lösung mit einem pH-Wert zwischen 6 und 9 dargereicht wird.

7. Verwendung nach einem der vorangegangenen Ansprüche 1 bis 6, zur Prävention und/oder zur Behandlung von Nesselausschlag, der von ein Brennen auslösenden Mitteln, ausgewählt aus der Gruppe insbesondere enthaltend Quallen, Brennesseln, Hippomanes mancincllae, Giflsumach und Insektenstiche, hervorgerufen wird
